# EUROPÄISCHE PATENTANMELDUNG

(11) **EP 2 703 022 A1**
(43) Veröffentlichungstag der Anmeldung: **05.03.2014**
(21) Anmeldenummer: 12182782.8
(22) Anmeldetag: 03.09.2012
(51) Int. Cl.: A61M 5/30

(54) **Einmalinjektor mit zweistufiger Auslösung**

(71) Anmelder: LTS LOHMANN Therapie-Systeme AG, 56626 Andernach (DE)
(72) Erfinder: Spilgies, Heiko, 81373 München (DE); Hadaschik, Roman, 99817 Eisenach (DE); Wortmann, Uwe, 35037 Marburg (DE); Heuser, Karsten, 53498 Bad Breisig (DE)

(57) **Zusammenfassung**

Die Erfindung betrifft einen Einweginjektor (4) mit einem Gehäuse (10) und einer, einen Energiespeicher (50), einen Betätigungsstempel (60) und eine Auslösehülse (90) umfassenden Auslösevorrichtung. Der Betätigungsstempel und das Gehäuse weisen zueinander gerichtete Blockierflächen (17, 72) auf, zwischen denen mindestens ein Verdrängungskörper (86) anordbar ist. Das Betätigungselement ist um die Längsachse des Einweginjektors schwenkbar und umfasst zu den Blockierflächen versetzt angeordnete Verdrängersegmente (73), so dass die Verdrängersegmente in Kontakt mit dem Verdrängungskörper bringbar sind. Außerdem ist mittels eines Verschiebens der Auslösehülse (90) in Richtung der Längsachse eine Wegbegrenzung des mittels des Verdrängersegments belasteten Verdrängerkörpers zur Freigabe des Energiespeichers aufhebbar.

Mit der vorliegenden Erfindung wird ein Einmalinjektor entwickelt, der auch nach langer Lagerzeitdauer ein sicheres Auslösen gewährt. Außerdem muß der Anwender den Einmalinjektor vor dem Auslösen bewußt entriegeln.

## Beschreibung

Die Erfindung betrifft einen Einweginjektor mit einem Gehäuse und einer, einen Energiespeicher, einen Betätigungsstempel und eine Auslösehülse umfassenden Auslösevorrichtung.

Aus der nachveröffentlichten DE 10 2007 031 630 A1 ist ein derartiger Einmalinjektor bekannt. Der Auslösemechanismus wird mittels ist während der gesamten Lagerzeit belastet. Die zweistufige Auslösung kann ohne Änderung der Griffposition am Einmalinjektor erfogen.

Der vorliegenden Erfindung liegt die Problemstellung zugrunde, einen Einmalinjektor zu entwickeln, der auch nach langer Lagerzeitdauer ein sicheres Auslösen gewährt. Außerdem soll der Anwender den Einmalinjektor vor dem Auslösen bewußt entriegeln.

Diese Problemstellung wird mit den Merkmalen des Hauptanspruchs gelöst. Dazu weisen der Betätigungsstempel und das Gehäuse zueinander gerichtete Blockierflächen auf, zwischen denen mindestens ein Verdrängungskörper anordbar ist. Das Betätigungselement ist um die Längsachse des Einweginjektors schwenkbar und umfasst zu den Blockierflächen versetzt angeordnete Verdrängersegmente, so dass die Verdrängersegmente in Kontakt mit dem Verdrängungskörper bringbar sind. Außerdem ist mittels eines Verschiebens der Auslösehülse in Richtung der Längsachse eine Wegbegrenzung des mittels des Verdrängersegments belasteten Verdrängerkörpers zur Freigabe des Energiespeichers aufhebbar

Weitere Einzelheiten der Erfindung ergeben sich aus den Unteransprüchen und den nachfolgenden Beschreibungen schematisch dargestellter Ausführungsbeispiele.
- Figur 1:: Einweginjektor im Lagerzustand;
- Figur 2:: Schnitt des Gehäuses in der Höhe der Durchbrüche;
- Figur 3:: Seitenansicht des Betätigungselements;
- Figur 4:: Ansicht des Betätigungselements von unten;
- Figur 5:: Draufsicht der Adapterhülse;
- Figur 6:: Detail des Einweginjektors aus Figur 1;
- Figur 7:: Detail aus Figur 6;
- Figur 8:: Detail des Einweginjektors nach dem Schwenken des Betätigungselements;
- Figur 9:: Detail des Einweginjektors nach der Betätigung des Auslöseelements;
- Figur 10:: Detail des Einweginjektors mit verdrängtem Verdrängungskörper;
- Figur 11:: Detail des ausgelösten Einweginjektors;
- Figur 12:: Detail der Auslösevorrichtung mit einem weitgehend quaderförmigen Verdrängungskörper.

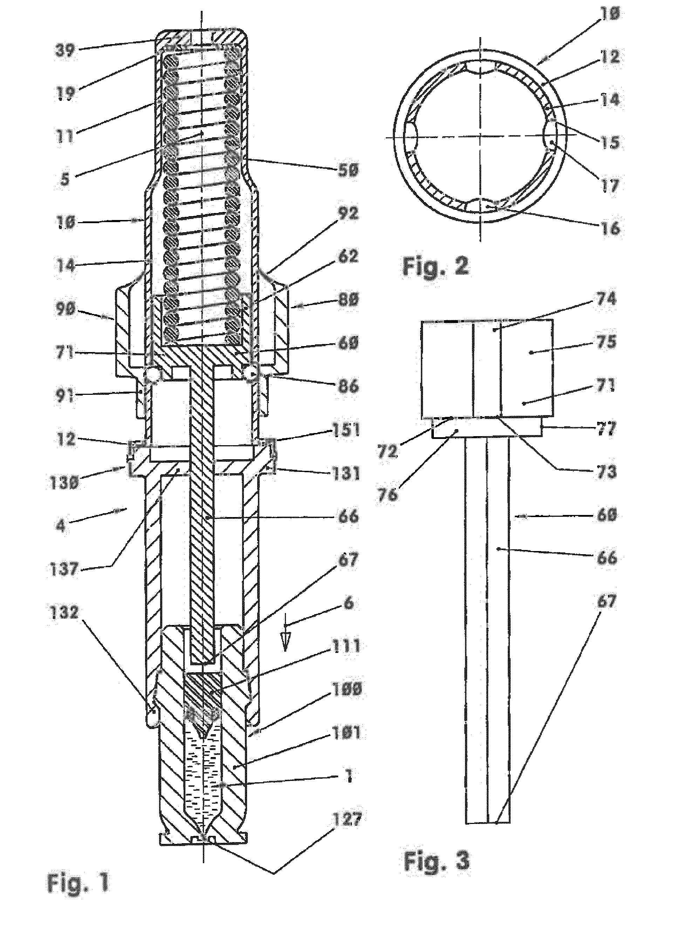

Die Figuren 1 - 11 zeigen einen Einweginjektor (4). Deartige Einmalinjektoren (4) werden zum einmaligen Einbringen beispielsweise einer definierten Dosis einer wirkstoffhaltigen Lösung in den menschlichen Körper eingesetzt. Die z.B. durch die Haut hindurch einzubringende Lösung (1) ist in einer Zylinder-Kolben-Einheit (100) gelagert. Nach dem Auslösen des Einweginjektors (4) wird der Kolben (111) dieser Zylinder-Kolben-Einheit (100) in Richtung der Austrittsöffnung (127) verschoben und dabei die flüssige Medikamentenlösung (1) durch eine Nadel hindurch oder nadellos unter die Haut verdrängt.

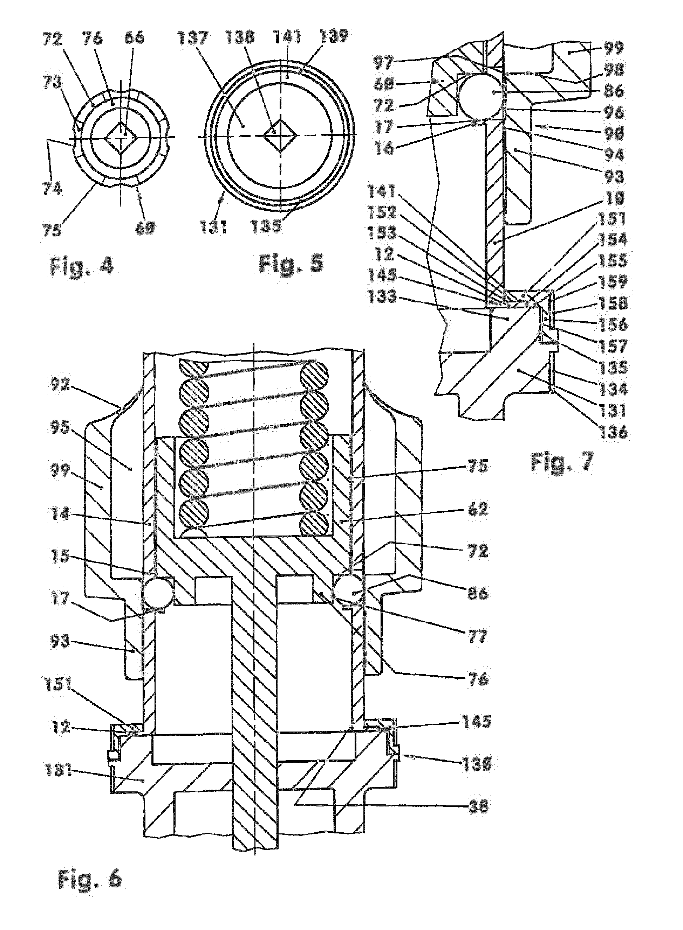

Der Einweginjektor (4) umfasst ein Gehäuse (10) und eine Auslösevorrichtung (80). Das Gehäuse (10) ist glockenartig aufgebaut. Auf der Innenseite des dem Benutzer zugewandten Bodens (39) stützt sich ein Energiespeicher (50) z.B. mittels einer Distanzscheibe (19) ab. Im Ausführungsbeispiel ist der Energiespeicher (50) ein Federelement, z.B. eine Druckfeder (50). Auch der Einsatz eines pyrotechnischen Energiespeichers (50) ist denkbar. Das aus einem austenitischen Stahlwerkstoff oder aus Kunststoff gefertigte Gehäuse (10) hat eine gestufte Wandung (11), die sich zu einer dem Boden (39) entfernten Öffnung (38) hin aufweitet, vgl. Figur 6. Diese Öffnung (38) ist von einem außenliegenden Ringflansch (12) umgeben. Die Ebene des Ringflanschs (12) ist normal zur Längsrichtung (5) des Einweginjektors (4) angeordnet. Die Breite des Ringflansches (12) in radialer Richtung ist größer oder gleich der Wandstärke des Gehäuses (10).

Im unteren Mantelbereich (14) hat das Gehäuse (10) beispielsweise vier gleichmäßig am Umfang verteilte Durchbrüche (15). Diese Durchbrüche (15) haben z.B. einen weitgehend quadratischen Querschnitt, wobei die Ecken abgerundet sein können. Das Gehäuse (10) kann auch einen, zwei oder drei Durchbrüche (15) aufweisen.

Die Figur 2 zeigt einen Querschnitt des Gehäuses (10) mit Blickrichtung auf den Ringflansch (12), wobei die Schnittebene die Durchbrüche (15) schneidet. An den Unterkanten der Durchbrüche (15) sind Stützelemente (16) nach innen gebogen. Diese sind beispielsweise kreissegmentförmig ausgebildet. Die dem Betrachter der Figur 2 zugewandten Flächen (17) dieser Stützelemente (16) werden im Folgenden als Blockierflächen (17) bezeichnet. Diese können in einer Ebene normal zur Längsrichtung (5) liegen. Sie können mit dieser Ebene aber auch - wie in der Figur 7 dargestellt - einen Winkel bis zu 10 Grad einschließen. Hierbei sind die innenliegenden Enden der Blockierflächen (17) näher an der Öffnung (38) angeordnet als die außenliegenden Enden.

Im Gehäuse (10) ist das Betätigungsteil (60) angeordnet. Dieses ist in einer Seitenansicht in der Figur 3 und in einer Ansicht von unten in der Figur 4 dargestellt. Das Betätigungsteil (60) umfasst einen Führungsring (62), einen Stempel (71) und eine Kolbenbetätigungsstange (66).

Der auf dem Stempel (71) sitzende Zentrierring (62) umgreift und führt im Ausführungsbeispiel die Feder (50) auf ihrer Außenseite. Auch eine Innenzentrierung und -führung der Feder (50) ist denkbar. Der Durchmesser des scheibenartigen Stempels (71) ist beispielsweise um zwei zehntel Millimeter kleiner als der Innendurchmesser des Gehäuses (10). Der Stempel (71) ist z.B. normal zur Längsrichtung (5) des Einweginjektors (4) ausgerichtet.

Die entgegengesetzt zum Zentrierring (62) angeordnete Unterseite des Betätigungsstempels (60) hat im Ausführungsbeispiel vier Blockierflächen (72) und vier versetzt zu diesen angeordnete Verdrängersegmente (73). Die Blockierflächen (72) sind in den Darstellungen der Figuren 1, 3, 6 und 7 in einer Ebene normal zur Längsachse (5) angeordnete Flächenabschnitte. Die bezogen auf die Längsachse (5) beispielsweise jeweils um einen Winkel von 45 Grad hierzu versetzt angeordneten Verdrängersegmente (73) weisen jeweils eine Ausnehmung (74) auf, die sich in der Mantelfläche (75) des Zentrierrings (62) fortsetzt. Jede der in die Ebene der Blockierflächen (72) projizierten zylindersegementförmigen Ausnehmungen (74) hat in dieser Ebene einen Flächeninhalt, der kongruent ist zu der in dieselbe Ebene projizierte Blockierfläche (17) des Gehäuses (10). Die projizierte Fläche einer Ausnehmung (74) kann auch größer sein als die projizierte Blockierfläche (17) des Gehäuses (10). Die einzelne Ausnehmung (74) kann auch ein Segment des Stempels (71) sein. Das einzelne Verdrängersegment (73) kann eine schiefe Ebene umfassen. Nach innen hin sind die Blockierflächen (72) und die Verdrängungssegmente (73) begrenzt durch einen Begrenzungsring (76). Dieser hat eine zylinderförmige Mantelfläche (77).

Die Kolbenbetätigungsstange (66), der sogenannte Kolbenschieber (66), ist entgegengesetzt zum Führungsring (62) zentrisch am Stempel (71) angeordnet. Im eingebauten Zustand durchdringt ihr auskragendes Ende (67) die Öffnung (38) des Gehäuses (10). Der Kolbenschieber (66) hat im Ausführungsbeispiel einen konstanten, quadratischen Querschnitt. Es ist aber auch denkbar, den Kolbenschieber (66) mit einem dreieckigen, sechseckigen, polygonalen, etc. Querschnitt auszubilden. Im eingebauten Zustand, vgl. Figur 1, ist das freie Ende (67) in den Zylinder (101) eingetaucht und endet wenige Millimeter oberhalb des Kolbens (111). Die Diagonale der Querschnittsfläche des Kolbenschiebers (66) ist kleiner als der Innendurchmesser des Zylinders (101). Die Betätigungsstange (66) kann somit im Zylinder (101) führbar sein.

Zwischen den Blockierflächen (17, 72) des Gehäuses (10) und des Betätigungsteils (60) sind in der Darstellung der Figur 1 Verdrängungskörper (86) angeordnet. Im dargestellten Ausfühungsbeispiel sind dies Kugeln (86), die sich auf beiden Blockierflächen (17, 72) abstützen. Die beiden Anlagepunkte sind im Ausführungsbeispiel übereinander angeordnet. Gegebenenfalls verhindert auch die die Durchbrüche (15) verschließende Auslösehülse (90) ein Auswandern der Kugeln (86). Bei einer von innen nach außen ansteigenden Gehäuse-Blockierfläche (17) werden die Kugeln (86) nach innen in Richtung des Begrenzungsrings (76) belastet. Der Radius der Verdrängungskörper (86) kann kleiner oder gleich dem gedachten Radius der Ausnehmungen (74) sein.

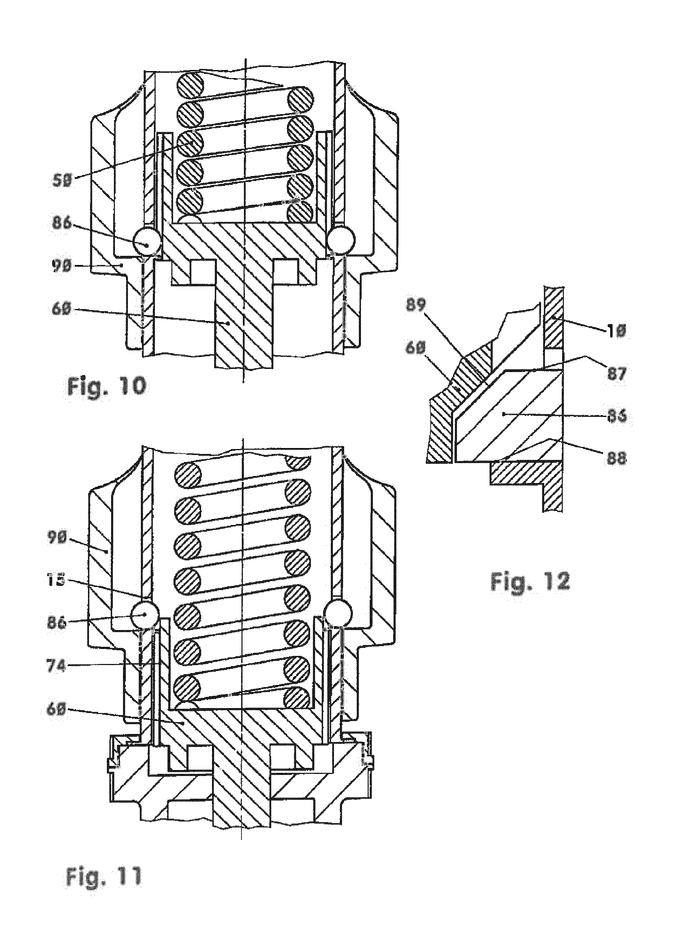

Die Verdrängungskörper (86) können auch im Querschnitt weitgehend keilförmig oder quaderförmig ausgebildet sein. Ein derartiger Körper ist beispielsweise in der Figur 12 dargestellt. Er umfasst zwei z.B. parallel zueinander angeordnete Sperrflächen (87, 88). Nach innen hin grenzt die obere Sperrfläche (87) an eine Verdrängerfläche (89). Letztere schließt mit einer Geraden parallel zur Längsrichtung (5) beispielsweise einen Winkel kleiner oder gleich 45 Grad ein.

Der Ringflansch (12) des Gehäuses (10) ist in einer Adapterbaugruppe (130) gehalten, vgl. die Figuren 6 und 7. Diese Adapterbaugruppe (130) umfasst eine Adapterhülse (131) und einen Adapterring (151). Beide Teile sind beispielsweise aus einem thermoplastischen Kunststoff hergestellt.

Die Apapterhülse (131) hat eine weitgehehend topfförmige Gestalt. An ihrem dem Energiespeicher (50) abgewandten Ende hat sie z.B. aufspreizbare Hintergriffselemente (132), die im montierten Zustand die Zylinder-Kolbeneinheit (100) halten. An ihrem dem Energiespeicher (50) zugewandten Ende hat die Adapterhülse (131) einen Stützring (133) mit einer planen Oberseite. Die Mantelfläche (134) des Stützrings (133) weist ein Außengewinde (135) auf. In der Darstellung der Figur 7 ist unterhalb dieses Gewindes (135) eine Schlüsselfläche (136) zum Ansetzen eines Montagewerkzeugs angeordnet.

Die Figur 5 zeigt eine Draufsicht der Adapterhülse (131). Die nach oben gerichtete Bodenscheibe (137) hat in dieser Darstellung einen quadratischen Durchbruch (138). Durch diesen Durchbruch (138) hindurch ist im montierten Zustand die Kolbenbetätigungsstange (66) geführt. Das Spiel der Betätigungsstange (66) im Durchbruch (138) ist beispielsweise kleiner als zwei zehntel Millimeter. Die Oberseite des Stützrings (133) umfasst eine außenliegende Preßfläche (139) und eine hierzu konzentrische, daran angrenzende innenliegende Gleitfläche (141).

Der Apapterring (151) hat einen kreisförmigen Durchbruch (152). Der Durchmesser des Durchbruchs (152) ist größer als der Außendurchmesser des Gehäuses (10) und kleiner als der Außendurchmesser des Ringflanschs (12). Die Unterseite des Adapterrings (151) umfasst eine axiale (153) und eine radiale Gleitfläche (154), die im montierten Zustand zusammen mit der Gleitfläche (141) der Adapterhülse (131) den Ringflansch (12) des Gehäuses (10) umgeben. Das radiale und das axiale Spiel des Gleitlagers (145) betragen jeweils beispielsweise ein zehntel Millimeter.

Außerhalb der Gleitfläche (153) des Adapterrings (151) ist ein Preßring (155) angeordnet. Dieser kontaktiert im eingebauten Zustand die Preßfläche (139) der Adapterhülse (131). Außerhalb des Preßrings (155) steht an der Unterseite des Adapterrings (151) ein Befestigungsring (156) hervor. Dieser hat ein zum Außengewinde (135) der Adapterhülse (131) komplementäres Innengewinde (157) und an seiner Mantelfläche (158) Schlüsselflächen (159). Bei der Montage können so die Adapterhülse (131) und der Adapterring (151) mit Vorspannung montiert werden.

Die Auslösehülse (90) ist ringförmig aufgebaut. Sie umgreift das Gehäuse (10) und ist an diesem geführt. Die gesamte Führungslänge kann größer oder gleich dem doppelten Gehäusedurchmesser sein. Die Auslösehülse (90) ist im Ausführungsbeispiel aus einem Zwei-Komponentenwerkstoff hergestellt. Sie hat in den Darstellungen der Figuren 1 und 6 einen untenliegenden Führungs- und Sperrring (91) und einen Wandbereich (99), die aus einem verformungssteifen Werkstoff hergestellt sind. Eine obenliegende Dichtlippe (92) ist beispielsweise aus einem elastisch verformbaren Werkstoff hergestellt. Beide Werkstoffe können thermoplastische Kunststoffe sein.

Zwischen der Dichtlippe (92) und dem Führungs- und Sperrring (91) ist ein mittels des Wandbereichs (99) begrenzter Aufnahmeraum (95) angeordnet. Die Ausdehnung des Aufnahmeraums (95) in radialer Richtung ist im montierten Zustand größer als der Durchmesser eines Verdrängungskörpers (86). Bei einem nicht kugelförmigen Verdrängungskörper (86) ist die radiale Länge des Aufnahmeraums (95) größer als die maximale Ausdehnung des Verdrängungskörpers (86) in dieser Richtung.

Der Führungs- und Sperrring (91) hat in den Darstellungen der Figuren 1, 6 und 7 einen untenliegenden Führungsabschnitt (93) mit zwei einwertigen Gleitlagern (94) und einen darüber angeordneten Sperrabschnitt (96). Der Sperrabschnitt (96) hat eine zylinderförmige Innenwandung und geht in einem Übergangsradius (97) in die Bodenfläche (98) des Aufnahmeraums (95) über.

Gegebenenfalls kann die Auslösehülse (90) im montierten Zustand federbelastet oder mittels einer Banderole an einem Verrutschen gehindert sein.

Bei der Montage des Einmalinjektor (4) werden beispielsweise zunächst der Energiespeicher (50) und der Betätigungsstempel (60) in das Gehäuse (10) eingesetzt. Hierbei werden die Aussparungen (74) des Betätigungsteils (60) entlang der Stützelemente (16) des Gehäuses (10) geführt. Nun wird von oben her der Adapterring (151) auf das Gehäuse (10) aufgesetzt. Von unten her wird die Adapterhülse (131) auf das Betätigungsteil (60) aufgeschoben. Beim Verschrauben der Gewinde (135, 157) des Adapterrings (151) und der Adapterhülse (131) werden der Preßring (155) und die Preßfläche (139) belastet. So kann in Abhängigkeit der Vorspannung der Schraubverbindung das Spiel des Gleitlagers (145) eingestellt werden und die Schraubverbindung gegen ein Lösen gesichert werden. Die Auslösehülse (90) wird von oben auf das Gehäuse (10) aufgeschoben.

Nun kann das Betätigungsteil (60) von seinem freien Ende (67) her relativ zum Gehäuse (10) und zur Adapterbaugruppe (130) eingeschoben werden, so dass die Feder (50) komprimiert wird. Hierbei wird der Energiespeicher (50) geladen. Die Adapterbaugruppe (130) kann nun z.B. um einen Winkel von 45 Grad relativ zum Gehäuse (10) geschwenkt werden. Bei diesem Schwenken nimmt die Adapterhülse (131) mittels des Formschlusses zwischen dem Durchbruch (138) und dem Kolbenschieber (66) das Betätigungsteil (60) mit. Die Blockierflächen (72) des Betätigungsteils (60) stehen nun oberhalb der Blockierflächen (17) des Gehäuses (10).

Nach dem Sichern der Bauteile in dieser Position werden die Verdrängungskörper (86) eingesetzt. Hierzu wird beispielsweise die Auslösehülse (90) in der Auslöserichtung (6) nach unten geschoben. Nach dem Aufbiegen der Dichtlippe (92) können die Kugeln (86) in den nun geöffneten Aufnahmeraum (95) eingeführt werden. Durch die Durchbrüche (15) hindurch werden die Verdrängungskörper (86) dann zwischen die Blockierflächen (17, 72) eingeschoben. Nun kann die temporäre Montagesicherung des Betätigungsteils (60) und der Feder (50) gelöst werden. Der federbelastete Stempel (71) presst die Kugeln (86) jetzt gegen die Blockierflächen (17) des Gehäuses (10). Der Einweginjektor (4) ist verriegelt. Die Auslösehülse (90) kann wieder nach oben geschoben werden und wird z.B. mittels eines Originalitätsverschlusses gesichert.

In die Adapterhülse (131) wird die befüllte Zylinder-Kolben-Einheit (100) eingesetzt und beispielsweise unlösbar verrastet.

Der Einweginjektor (4) kann - mit oder ohne eingebauter Zylinder-Kolben-Einheit (100) - mehrere Monate oder Jahre gelagert werden.

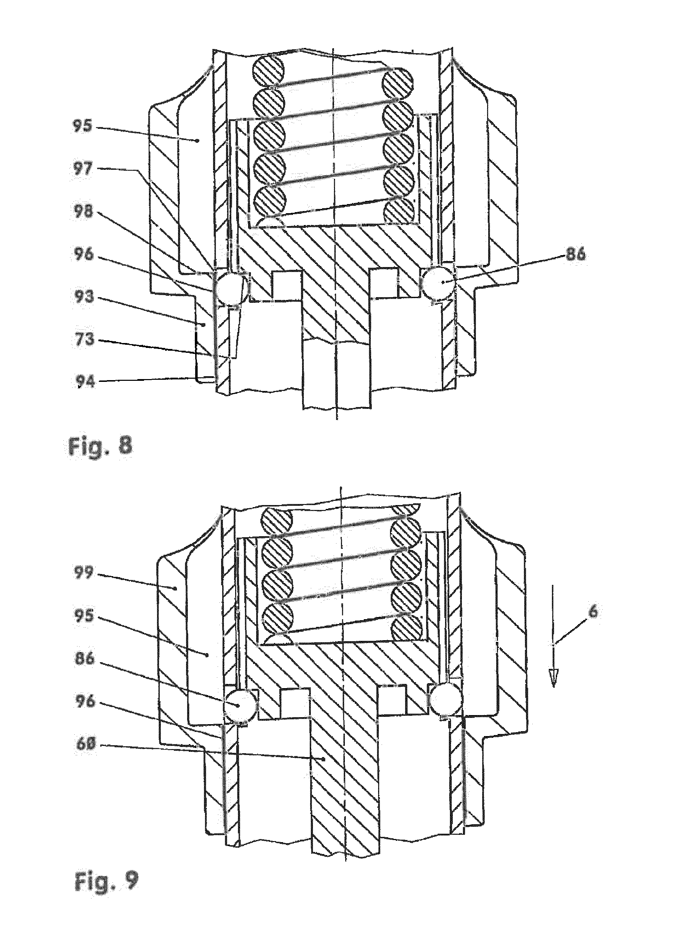

Um den Einmalinjektor (4) einzusetzen, hält der Arzt oder Patient den Injektor (4) mit zwei Händen und verdreht die Adapterbaugruppe (130) relativ zum Gehäuse (10). Mit dem Verschwenken der Adapterbaugruppe (130) wird der Betätigungsstempel (60) über die formschlüssige Verbindung (66, 138) mitgenommen. Das Gehäuse (10) schwenkt relativ zur Adapterbaugruppe (130) im Gleitlager (145). Die Durchbrüche (15) und/oder die Getalt der Stützelemente (16) verhindern ein Auswandern der Kugeln (86). Die Verdrängersegmente (73) kommen in Kontakt mit den Verdrängerkörpern (86), vgl. Figur 8. Hierbei wird die Feder (50) um wenige zehntel Millimeter entlastet, so dass der Einmalinjektor (4) in dieser Position verrastet. Ein Weiterschwenken der Adapterbaugruppe (130) und des Batätigungsstempels (60) ist nicht möglich.

Der Kraftangriff an der Kugel (86) hat sich nach innen verlagert, so dass die Kugel (86) nach außen in Richtung des Sperrabschnitts (96) der Auslösehülse (90) verdrängt wird. Die Kugel (86) stößt an den Sperrabschnitt (96) an, der ein weiteres Verdrängen der Kugel (86) verhindert. Hiermit ist die erste Sicherung des zweifufigen Auslösemechanismus aufgehoben.

Nach dem Ansetzen des Einweginjektors (4) an der Haut des Patienten kann der Injektor (4) ausgelöst werden. Hierzu wird die Auslösehülse (90) in der Auslöserichtung (6), in der Darstellung der Figur 1 nach unten, geschoben. Die Wegbegrenzung des Verdrängungskörpers (86) ist aufgehoben, vgl. Figur 9. Der in der Figur 9 dargestellte Zustand ist nicht stabil, denn nach der Aufhebung der Sperre verdrängt der sich entladende Energiespeicher (50) mittels des Stempels (71) die Kugel (86) nach außen in den Aufnahmeraum (95), vgl. Figur 10.

Das beschleunigte Betätigungsteil (60) trifft mit der Betätigungsstange (66) auf den Kolben (111) der Zylinder-Kolben-Einheit (100). Der in Richtung der Austrittöffnung (127) verschobene Kolben (111) verdrängt die gelagerte Medikamentenlösung (1) durch die Austrittsöffnung (127) hindurch in die Haut des Patienten.

Der Betätigungsstempel (60) verfährt hierbei relativ zu dem im Gehäuse (10) und/oder im Aufnahmeraum (95) liegenden Verdrängungskörper (86). Gegebenenfalls kann der Verdrängungskörper (86) auch in die Aussparungen (74) des Stempels (71) hineinragen.

Sobald der Kolben (111) und das Betätigungsteil (60) in der vorderen Endposition stehen, kann die Kugel (86) oberhalb des Führungsrings (62) liegen. Wird die Auslösehülse (90) jetzt wieder nach oben gezogen, kann die Kugel (86) z.B. mittels einer schrägen Bodenfläche (98) in das Gehäuse (10) verdrängt werden. Der Verdrängungskörper (86) kann dann ein erneutes Einschieben des Betätigungselements (60) in das Gehäuse (10) blockieren. Eine nochmalige Verwendung des Einmalinjektors (4) ist damit wirksam verhindert.

Selbstverständlich ist es auch denkbar, die verschiedenen genannten Ausführungsformen miteinander zu kombinieren.

### Bezugszeichenliste:

- 1: Medikamentenlösung
- 4: Einweginjektor, Einmalinjektor
- 5: Mittellinie des Injektors, Längsrichtung
- 6: Auslösebewegungsrichtung, Abwärtsbewegung Richtungspfeil

- 10: Gehäuse
- 11: Wandung
- 12: Ringflansch

- 14: Mantelbereich
- 15: Durchbrüche
- 16: Stützelemente
- 17: Flächen, Blockierflächen

- 19: Distanzscheibe

- 38: Öffnung
- 39: Boden

- 50: Energiespeicher, Federelement, Druckfeder

- 60: Betätigungsteil, Betätigungsstempel, Betätigungselement
- 62: Führungsring, Zentrierring, Führungselement für (50)

- 66: Kolbenbetätigungsstange, Kolbenschieber
- 67: auskragendes Ende von (66), freies Ende von (66)

- 71: Stempel
- 72: Blockierflächen
- 73: Verdrängersegmente
- 74: Ausnehmungen, Aussparungen
- 75: Mantelfläche von (71)
- 76: Begrenzungsring
- 77: Mantelfläche von (76)

- 80: Auslösevorrichtung

- 86: Verdrängungskörper, Kugeln
- 87: Sperrfläche
- 88: Sperrfläche
- 89: Verdrängerfläche

- 90: Auslösehülse
- 91: Führungs- und Sprengring
- 92: Dichtlippe
- 93: Führungsabschnitt
- 94: Gleitlager
- 95: Aufnahmeraum
- 96: Sperrabschnitt
- 97: Übergangsradius
- 98: Bodenfläche
- 99: Wandbereich

- 100: Zylinder-Kolben-Einheit
- 101: Zylinder

- 111: Kolben

- 127: Austrittsöffnung

- 130: Adapterbaugruppe
- 131: Adapterhülse
- 132: Hintergriffselemente
- 133: Stützring
- 134: Mantelfläche
- 135: Außengewinde
- 136: Schlüsselfläche
- 137: Bodenscheibe
- 138: Durchbruch
- 139: Preßfläche

- 141: Gleitfläche

- 145: Gleitlager

- 151: Adapterring
- 152: Durchbruch
- 153: axiale Gleitfläche
- 154: radiale Gleitfläche
- 155: Preßring
- 156: Befestigungsring
- 157: Innengewinde
- 158: Mantelfläche
- 159: Schlüsselflächen

## Patentansprüche

1. Einweginjektor (4) mit einem Gehäuse (10) und einer, einen Energiespeicher (50), einen Betätigungsstempel (60) und eine Auslösehülse (90) umfassenden Auslösevorrichtung (80), **dadurch gekennzeichnet,**
- **dass** der Betätigungsstempel (60) und das Gehäuse (10) zueinander gerichtete Blockierflächen (17, 72) aufweisen, zwischen denen mindestens ein Verdrängungskörper (86) anordbar ist,
- **dass** der Betätigungsstempel (60) um die Längsachse (5) des Einweginjektors (4) schwenkbar ist und zu den Blockierflächen (17, 72) versetzt angeordnete Verdrängersegmente (73) umfasst, so dass die Verdrängersegmente (73) in Kontakt mit dem Verdrängungskörper (86) bringbar sind und
- **dass** mittels eines Verschiebens der Auslösehülse (90) in Richtung der Längsachse (5) eine Wegbegrenzung des mittels des Verdrängersegments (73) belasteten Verdrängerkörpers (86) zur Freigabe des Energiespeichers (50) aufhebbar ist.

2. Einweginjektor (4) nach Anspruch 1, **dadurch gekennzeichnet, dass** er eine formschlüssig mit dem Betätigungsstempel (60) verbundene Adapterbaugruppe (130) zur Aufnahme einer Zylinder-Kolben-Einheit (100) umfasst.

3. Einweginjektor (4) nach Anspruch 2, **dadurch gekennzeichnet, dass** die Adapterbaugruppe (130) relativ zum Gehäuse (10) schwenkbar ist.

4. Einwegweginjektor (4) nach Anspruch 1, **dadurch gekennzeichnet, dass** der Energiespeicher (50) eine Druckfeder umfasst.

5. Einweginjektor (4) nach Anspruch 1, **dadurch gekennzeichnet, dass** der Betätigungsstempel (60) ein Führungselement (62) für den Energiespeicher (50) umfasst.

6. Einweginjektor (4) nach Anspruch 1, **dadurch gekennzeichnet, dass** die Verdrängersegmente (73) des Betätigungsstempels (60) Aussparungen (74) aufweisen, wobei die Flächen der Aussparungen (74) in einer Normalenebene zur Längsrichtung (5) kongruent sind zu den oder größer sind als die Blockierflächen (17) des Gehäuses (10) in einer parallelen Ebene.

7. Einweginjektor (4) nach Anspruch 1, **dadurch gekennzeichnet, dass** die Auslösehülse (90) einen öffenbaren Aufnahmeraum (95) umfasst.
